# EUROPEAN PATENT APPLICATION

(11) **EP 2 638 853 A1**
(43) Date of publication of application: **18.09.2013**
(21) Application number: 13158722.2
(22) Date of filing: 12.03.2013
(51) Int. Cl.: A61B 5/00, G06T 19/20

(54) **Selectably transparent electrophysiology map**

(30) Priority: 13.03.2012 US 201213418758
(71) Applicant: Biosense Webster (Israel), Ltd., Yokneam 20692 (IL)
(72) Inventor: Massarwa, Fady, 30100 Baka El Gharbiya (IL); Ilan, Ido, 20692 Yokneam (IL)
(74) Representative: Small, Gary James

(57) **Abstract**

A method for mapping a body organ, including receiving a three-dimensional (3D) map of the body organ together with items of auxiliary information having respective location coordinates in a frame of reference of the 3D map and apportioning the items into a plurality of sub-groups. The method further includes assigning to a selected sub-group a visibility parameter indicative of a relative visibility of the selected sub-group in relation to the map and to other sub-groups. The method also includes displaying the 3D map of the body organ in a selected orientation while selectively superimposing on the 3D map one or more of the items in the selected sub-group responsively to the orientation, the respective location coordinates of the items, and the assigned visibility parameter.

## Description

### FIELD OF THE INVENTION

The present invention relates generally to graphic displays, and specifically to displaying of electrophysiological data in a map.

### BACKGROUND OF THE INVENTION

In medical procedures, such as mapping the electrical activity of the heart, there is typically a large amount of information that may be presented to a professional performing the mapping, and/or performing a procedure using the mapping. The large amount of information presented may lead to difficulties in comprehension of the information. A system to improve the comprehension of the information would be beneficial.

### SUMMARY OF THE INVENTION

There is provided, according to an embodiment of the present invention, a method for mapping a body organ, including:
receiving a three-dimensional (3D) map of the body organ together with items of auxiliary information having respective location coordinates in a frame of reference of the 3D map;
apportioning the items into a plurality of sub-groups;
assigning to a selected sub-group a visibility parameter indicative of a relative visibility of the selected sub-group in relation to the map and to other sub-groups; and
displaying the 3D map of the body organ in a selected orientation while selectively superimposing on the 3D map one or more of the items in the selected sub-group responsively to the orientation, the respective location coordinates of the items, and the assigned visibility parameter.

Typically, the items of auxiliary information include a further 3D map of a portion of the body organ, and the further 3D map is assigned a further-3D-map visibility parameter. The further-3D-map visibility parameter may cause the further 3D map to be locally transparent, so that all elements of the further 3D map are visible while the further 3D map is opaque with respect to the 3D map.

In an embodiment the further 3D map is disjoint from the 3D map.

In an alternative embodiment the further 3D map intersects the 3D map.

The body organ may include a heart, and the selected sub-group may include local activation times (LATs) of the heart. Typically, the LATs include measured LATs, and the LATs may include interpolated LATs derived from the measured LATs.

In a further alternative embodiment the relative visibility includes a transparency of the selected sub-group.

In a yet further alternative embodiment the relative visibility includes at least one of a color and a shading applied to the selected sub-group.

Typically, the sub-groups are selected from a set consisting of an ablation site, a catheter type, and a catheter measurement.

The relative visibility of an element in the selected sub-group may be a function of the location coordinates of the element. Alternatively or additionally, the relative visibility of an element in the selected sub-group may be a function of a proximity of the element to another element in the sub-group.

In a disclosed embodiment the relative visibility of an element in the selected sub-group is a function of a proximity of the element to another element in the other sub-groups.

In another disclosed embodiment the relative visibility of an element in the selected sub-group is a function of a time of the mapping of the body organ.

There is further provided, according to an embodiment of the present invention, apparatus for mapping a body organ, including:
a processor which is configured to:
   receive a three-dimensional (3D) map of the body organ together with items of auxiliary information having respective location coordinates in a frame of reference of the 3D map,
   apportion the items into a plurality of sub-groups, and
   assign to a selected sub-group a visibility parameter indicative of a relative visibility of the selected sub-group in relation to the map and to other sub-groups; and
   a screen, coupled to the processor, which is configured to display the 3D map of the body organ in a selected orientation while the processor selectively superimposes on the 3D map one or more of the items in the selected sub-group responsively to the orientation, the respective location coordinates of the items, and the assigned visibility parameter.

The present disclosure will be more fully understood from the following detailed description of the embodiments thereof, taken together with the drawings, in which:

### BRIEF DESCRIPTION OF THE DRAWINGS

Fig. 1 is a schematic illustration of a physiological mapping system, according to an embodiment of the present invention;
Figs. 2 and 3 are schematic illustrations of typical three-dimensional charts that may be presented on a screen of the system of Fig. 1, according to embodiments of the present invention;
Fig. 4 is a flowchart of steps performed for mapping a body organ such as a heart, according to an embodiment of the present invention; and
Fig. 5 and Fig. 6 are schematic examples of charts produced by following the steps of the flowchart, according to an embodiment of the present invention.

### DETAILED DESCRIPTION OF EMBODIMENTS

### OVERVIEW

An embodiment of the present invention provides a method and system for mapping a body organ, by selectively changing the relative visibility of elements of a chart of the body organ, as imaged on a screen. The imaged body organ, typically the heart of a patient, is presented in a three-dimensional (3D) format, and comprises a map of the organ upon which are superimposed one or more items of auxiliary information. The items of auxiliary information are classified into sub-groups, and one or more sub-groups are assigned respective visibility parameters which are indicative of respective relative visibilities of the sub-group. Sub-groups of the items may comprise, for example, location coordinates of points on a surface of the organ, measurements made on regions of the surface, actions performed on the regions, and types of instruments such as catheters associated with the body organ.

The chart of the body organ, comprising the map and the sub-groups of items, may be displayed on the screen in a selected orientation. The display superimposes on the 3D map one or more selected sub-groups responsively to the selected orientation, respective location coordinates of the one or more selected sub-groups, and assigned values of the respective visibility parameters of the one or more selected sub-groups.

By implementing selective relative visibilities of elements of the chart displayed on the screen, embodiments of the present invention considerably improve comprehension of the chart.

### SYSTEM DESCRIPTION

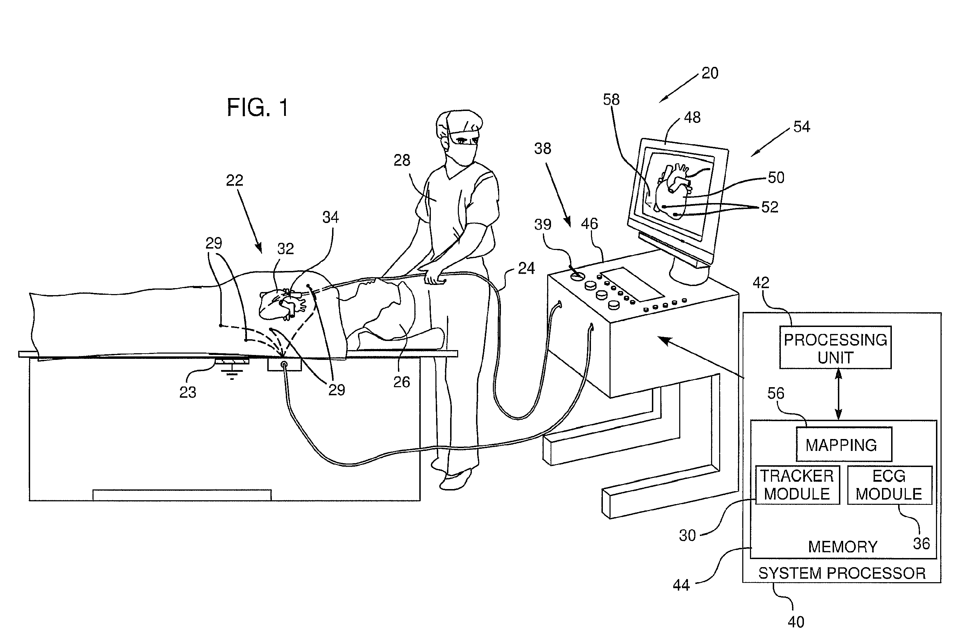

Reference is now made to Fig. 1, which is a schematic illustration of a physiological mapping system 20, according to an embodiment of the present invention. System 20 may be configured to map substantially any physiological parameter or combinations of such parameters. In the description herein, examples of mapped parameters are assumed to comprise local activation times (LATs) derived from intra-cardiac electrocardiogram (ECG) potential-time relationships. The measurement and use of LATs are well known in the electrophysiological arts. System 20 may map other physiological parameters, such as the location and/or size of cardiac lesions, the force applied to a region of the heart wall by a catheter, and the temperature of the heart wall region.

For simplicity and clarity, the following description, except where otherwise stated, assumes an investigative procedure wherein system 20 senses electrical signals from a heart 34, using a probe 24. A distal end 32 of the probe is assumed to have an electrode 22 for sensing the signals. Those having ordinary skill in the art will be able to adapt the description for multiple probes that may have one or more electrodes, as well as for signals produced by organs other than a heart.

Typically, probe 24 comprises a catheter which is inserted into the body of a subject 26 during a mapping procedure performed by a user 28 of system 20. In the description herein user 28 is assumed, by way of example, to be a medical professional. During the procedure subject 26 is assumed to be attached to a grounding electrode 23. In addition, electrodes 29 are assumed to be attached to the skin of subject 26, in the region of heart 34.

System 20 may be controlled by a system processor 40, comprising a processing unit 42 communicating with a memory 44. Processor 40 is typically mounted in a console 46, which comprises operating controls 38, typically including a pointing device 39 such as a mouse or trackball, that professional 28 uses to interact with the processor. Results of the operations performed by processor 40 are provided to the professional on a screen 48. The screen displays a three-dimensional (3D) map 50 of heart 34, together with items 52 of auxiliary information related to the heart and superimposed on the map, while the heart is being investigated. In the description and in the claims, an item of auxiliary information comprises any property or element that is, or that can be, associated with a region of the organ under consideration. In the examples described herein, the organ comprises heart 34. Examples of items 52 are provided below.

The combination of map 50 and items 52 is herein termed a chart 54 of the heart. Chart 54, comprising map 50 and items 52, is typically drawn on screen 48 relative to a frame of reference 58 of the map, and professional 28 is able to use pointing device 39 to vary parameters of the frame of reference, so as to display the chart in a selected orientation and/or at a selected magnification.

In addition to being able to have its orientation and magnification selected, chart 54, and its constituent parts: map 50 and items 52, may be presented on screen 48 in a number of different forms described below. In the description herein different forms of the chart and its parts are differentiated by having a letter, or a letter and a number, appended to the identifying numerals 50, 52, and 54. The different charts, maps and items are respectively referred to generically as charts 54, maps 50, and items 52.

Screen 48 typically also presents a graphic user interface to the user, and/or a visual representation of the ECG signals sensed by electrode 22.

Processor 40 uses software, including a probe tracker module 30 and an ECG module 36, stored in memory 44, to operate system 20. The software may be downloaded to processor 40 in electronic form, over a network, for example, or it may, alternatively or additionally, be provided and/or stored on non-transitory tangible media, such as magnetic, optical, or electronic memory.

ECG module 36 is coupled to receive electrical signals from electrode 22 and electrodes 29. The module is configured to analyze the signals and may present the results of the analysis in a standard ECG format, typically a graphical representation moving with time, on screen 48.

Probe tracker module 30 tracks sections of probe 24 while the probe is within subject 26. The tracker module typically tracks both the location and orientation of distal end 32 of probe 24, within the heart of subject 26. In some embodiments module 30 tracks other sections of the probe. The tracker module may use any method for tracking probes known in the art. For example, module 30 may operate magnetic field transmitters in the vicinity of the subject, so that magnetic fields from the transmitters interact with tracking coils located in sections of the probe being tracked. The coils interacting with the magnetic fields generate signals which are transmitted to the module, and the module analyzes the signals to determine a location and orientation of the coils. (For simplicity such coils and transmitters are not shown in Fig. 1.) The Carto® system produced by Biosense Webster, of Diamond Bar, CA, uses such a tracking method. Alternatively or additionally, tracker module 30 may track probe 24 by measuring impedances between electrode 23, electrodes 29 and electrodes 22, as well as the impedances to other electrodes which may be located on the probe. (In this case electrodes 22 and/or electrodes 29 may provide both ECG and tracking signals.) The Carto3® system produced by Biosense Webster uses both magnetic field transmitters and impedance measurements for tracking.

Using tracker module 30 processor 40 is able to measure locations of distal end 32, and form location coordinates of the locations in frame of reference 58 for construction of map 50. The location coordinates are assumed to be stored in a mapping module 56. In addition, mapping module 56 is assumed to store location coordinates of items 52 of auxiliary information associated with heart 34, and the procedure being performed on the heart.

Examples of items 52 and associated information of the items that mapping module 56 is able to store, include, but are not limited to, those given in Table I below. For each item 52, mapping module 56 stores, as appropriate, location coordinates associated with the item.

**Table I**

| **Item** | **Examples of Auxiliary information associated with item** |
|---|---|
| Local activation time (LAT) | Time at which a cardiac activation wave arrives at the LAT location |
| Ablation site | power dissipated, force applied, temperature reached, time at site |
| Catheter type | straight, lasso, multi-electrode, multi-prong; |
| Catheter measurement | potential (at catheter electrode or electrodes); force; temperature; rate of irrigation; energy, such as X-ray or ultrasound energy, flux. |

Tracker module 30 measures location coordinates for all items 52. Other modules in processor 40 measure auxiliary information associated with specific items 52. For example, ECG module 36 measures LATs. For clarity and simplicity, other modules measuring the auxiliary information, such as force, temperature, irrigation rate and energy flux modules, are not shown in Fig. 1.

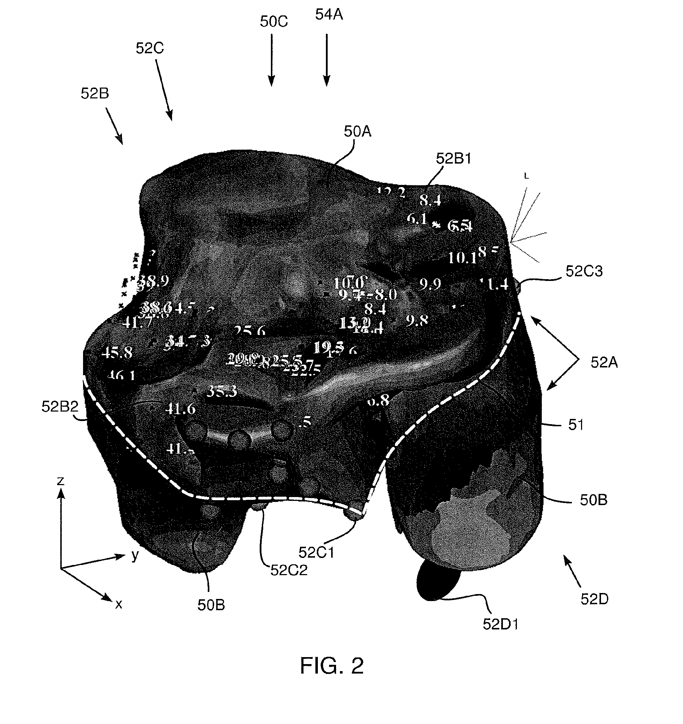

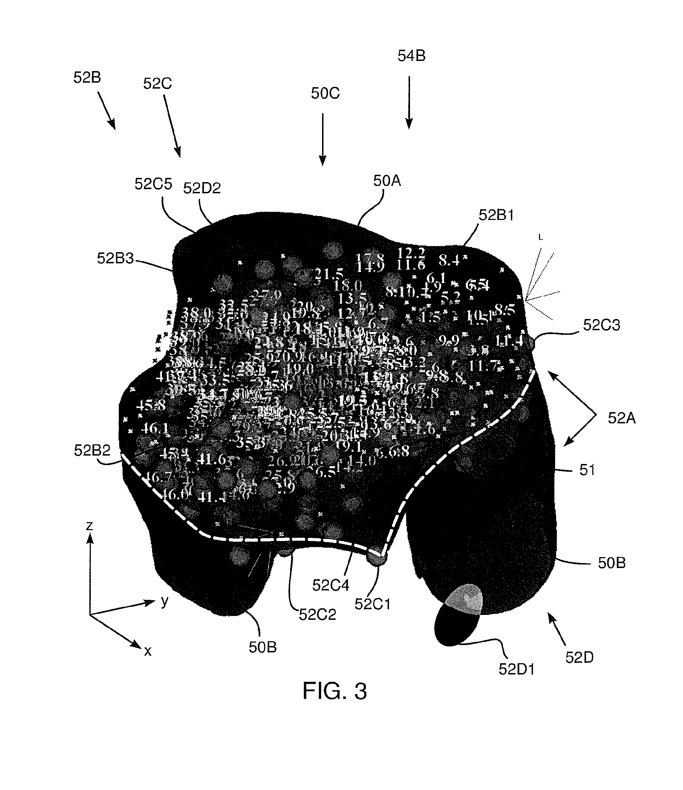

Figs. 2 and 3 are schematic illustrations of typical 3D charts that may be presented on screen 48, according to embodiments of the present invention. In the disclosure, charts are drawn on sets of xyz orthogonal axes. The illustrations of Figs. 2 and 3 are herein shown as gray-scale images, whereas typically the images are presented on screen 48 as color images.

In Fig. 2, a chart 54A illustrates parameters of a section of the heart that are drawn assuming that the heart is completely opaque, i.e., that the walls of the heart are non-transparent. Chart 54A is based on a first 3D map 50A of the walls of the section being illustrated, the first 3D map being constructed from measured location coordinates of points on the walls. Typically, to construct the first 3D map, a mesh of the measured points is produced, and 3D location coordinates of points between the measured points are determined by interpolation. The location coordinates of the measured and interpolated points are then used to produce a 3D continuous surface which is represented by 3D map 50A.

By way of example, first 3D map 50A is registered with a second 3D map 50B of the section. Map 50B is typically produced in a substantially similar manner to the method used for producing the first 3D map. However, this is not a requirement, so that in some embodiments the two maps may be produced from different sources. For example, one of the maps may be produced using magnetic resonance imaging (MRI) or by computerized tomography (CT).

In the embodiment described herein, the two maps are assumed to intersect so that part of map 50B covers 50A. An approximate intersection of the two maps is illustrated by a broken line 51. Nevertheless, there is no need that the two maps intersect, and in some embodiments the two maps have no intersection whatsoever, i.e., they are disjoint. Furthermore, in some embodiments one of the disjoint maps may enclose the other map.

The two registered maps are herein referred to as a combined 3D map 50C. In Fig. 2 both maps 50A and 50B are configured to be completely opaque, so that in combined 3D map 50C map 50B and only part of map 50A are visible.

Superimposed on combined map 50C are selected items 52 of auxiliary information, so as to form chart 54A. By way of example, items 52 that have been superimposed are:
● Items 52A, comprising estimated values of the LATs at location coordinates of the walls. Items 52A are herein also termed estimated LATs 52A. The superposition of estimated LATs 52A on map 50A is implemented by applying a gray scale according to the value of the estimated LAT, each level of gray corresponding to a numerical value of the estimated LAT.
● Items 52B, comprising measured values of the LATs at respective location coordinates of the walls, and herein also termed measured LATs 52B. Measured LATs 52B are superimposed on map 50C by incorporating the respective LAT numerical measured value in proximity to a respective point, representative of the location coordinate where the LAT is measured into the map. By way of example, specific measured values 52B1 and 52B2 of LATs are indicated in Fig. 2.
● Items 52C, comprising sites having information related to the procedure being performed, and herein drawn as spheres. Different types of information may be denoted by the size and/or color of the spheres. For example, red spheres may denote ablation sites, and a yellow sphere may denote the site of the His bundle. For simplicity, in the disclosure items 52C are assumed to be sites at which ablation has been performed, and are herein termed ablation sites 52C. Some of ablation sites 52C are only partially visible because of the opacity of map 50C. By way of example, specific ablation sites 52C1, 52C2, 52C3, the latter two of which are partially obscured by opaque portions of map 50A, are shown in Fig. 2.
● Items 52D, herein termed icons 52D and comprising icons representing the locations of distal ends of catheters being used during a procedure on the heart. In Fig. 2, while more than one catheter distal end may be present, only one distal end icon 52D1, of a lasso catheter, is visible. In the figure lasso catheter distal end icon 52D1 is only partly shown because of the opacity of map 50C.

In Fig. 3, a chart 54B illustrates similar parameters to the section of the heart shown in Fig. 2. Thus, as for chart 54A, chart 54B is based on the intersection of first 3D map 50A and second 3D map 50B, to form combined 3D map 50C. However, in contrast to chart 54A, chart 54B assumes that both the first and the second maps are transparent, so that all parts of both maps are visible.

As for chart 54A, estimated LATs 52A, measured LATs 52B, ablation sites 52C, and icons 52D are superimposed on chart 54B. Because of the transparency of both maps, all items that are visible in chart 54A are also visible in chart 54B. In addition, because of the transparency, in chart 54B further estimated LATs 52A, measured LATs 52B, ablation sites 52C, and all icons 52D are visible. For example, measured LAT 52B3, ablation sites 52C4, 52C5, and a multi-probe catheter distal end icon 52D2 are now visible. In addition, parts of elements that were not visible in chart 64A, such as ablation sites 52C2, 52C3, and icon 52D1, are now shown.

Comparison of Figs. 2 and 3 shows that for chart 54A the information presented is relatively clear, but that there may be missing information. Conversely, in chart 54B while there may be no missing information, the information presented is extremely cluttered and "noisy."

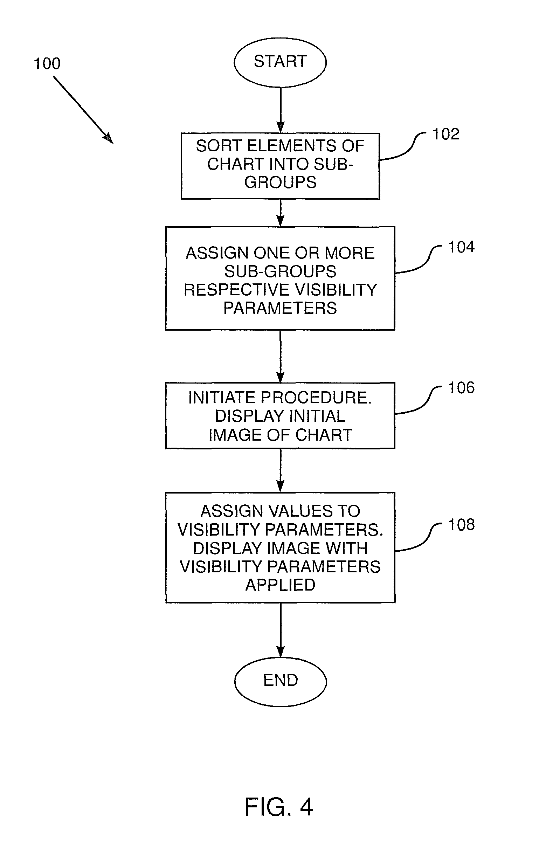

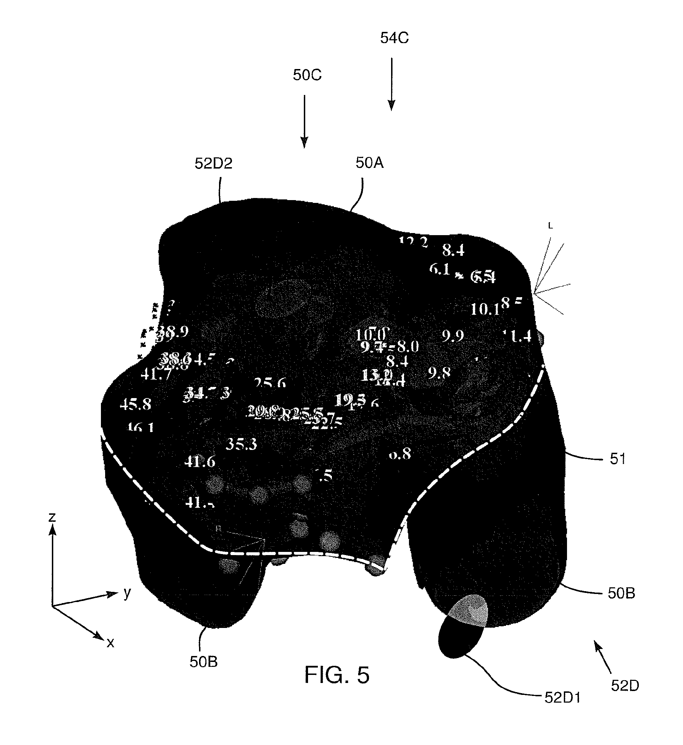

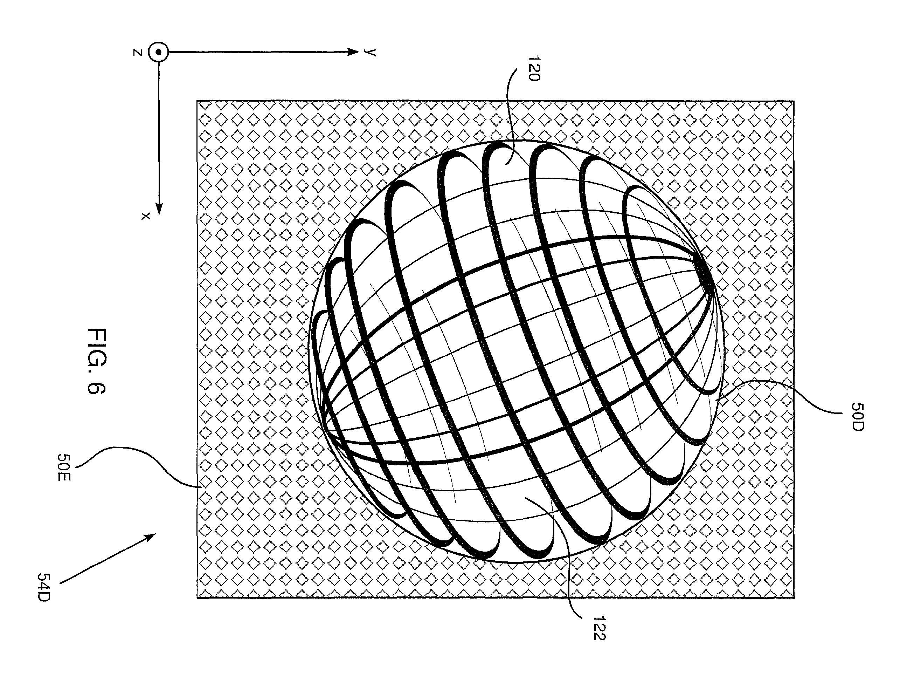

Fig. 4 is a flowchart 100 of steps performed for mapping a body organ such as heart 34, and Figs. 5 and 6 are schematic examples of charts produced by following the steps of the flowchart, according to embodiments of the present invention.

In a definition step 102, elements of a chart that is to be displayed on screen 48 are apportioned and classified into sub-groups. Referring back to Figs. 2 and 3, sub-groups of a chart are assumed to comprise one or more maps of the body organ. The sub-groups also comprise items of auxiliary information such as those exemplified above in Table I.

In a visibility step 104, at least one sub-group generated in step 102 is assigned a respective visibility parameter, a value of which is applied to elements of the sub-group. Typically, two or more sub-groups are each assigned visibility parameters. For a selected sub-group, the value of its visibility parameter determines a relative visibility of the sub-group in relation to the other sub-groups, including the map or maps of the displayed chart. The relative visibility comprises one or more visual characteristics, such as a transparency, of the sub-group.

In some embodiments the visibility parameter may also determine other visual characteristics of the sub-group, such as a color or shading to be applied to the sub-group. Typically, although not necessarily, all elements of a given sub-group are assigned the same visibility parameter. However, in some embodiments, the visibility parameter for an element of a given sub-group may be a function of factors of the element other than its membership in the given sub-group. For example, the visibility parameter of an element may be a function of its location coordinates, and/or its proximity to elements of the same or of another sub-group.

In a disclosed embodiment a given sub-group may comprise one given map used in the mapping, and all elements associated with the given map. In this case the visibility parameter may be assigned to the given map and its associated elements.

In an optional display step 106, typically implemented at the start of a procedure, a chart comprising all the elements of all the sub-groups is displayed on screen 48. Elements of sub-groups that have not had visibility parameters assigned are rendered visible. For sub-groups that have been assigned visibility parameters, the values of the parameters are set so that all the elements of these sub-groups are initially at least partially visible.

In a filter selection step 108, for each sub-group having an assigned visibility parameter, professional 28 assigns values for the visibility parameters until a desired visibility of each element of each sub-group is achieved. The assignment may be via professional 28 interacting with a graphic user interface on screen 48, using pointing device 39, or by any other convenient type of interaction. The chart displays on screen 48 according the relative visibility that has been set for each element. The chart, and the elements of its constituent sub-groups, is also displayed according to an orientation selected for the chart by professional 28, as well as according to the location coordinates of each of the elements of the chart.

As an example of the implementation of filter step 108, in the disclosed embodiment referred to above the visibility parameter assigned to the given sub-group may cause elements of the sub-group to be "locally" transparent. In the disclosure and in the claims, the phrase "locally transparent" as applied to a given map is to be understood as meaning that the given map and its associated elements may be considered to be mounted on a transparent surface, so that all features of the map, as well as its elements are visible. However, the locally transparent visibility parameter prevents the transparency extending beyond the given map, so that with respect to other maps in a chart, the given map is opaque.

Thus, if there is a second map behind the given map, the only features of the second map that are visible are those which are not shadowed by the given map. In other words the transparent characteristic of the given map does not apply from the point of view of the second map. Rather, as described above, with respect to the second map, the given map is opaque.

Fig. 5 illustrates a first application of flowchart 100 to produce a chart 54C. In chart 54C map 50A has had its relative visibility set so that the map is opaque, and map 50B has been set so that it is transparent. In addition, elements of a sub-group of catheter type items have had respective relative visibilities set according to the types of catheter in the sub-group, so that multi-probe catheters are visible. Thus icon 52D2 shows in chart 54C.

Fig. 6 illustrates a second application of flowchart 100 to produce a chart 54D. For clarity, maps in chart 54D are assumed to be simple geometrical shapes. In chart 54D a map 50D is a sphere, and a map 50E is a plane, parallel to the xy plane, which has been tessellated with diamond shapes. The plane is behind and disjoint from the sphere, so that the z values of all points on the sphere are greater than the z value of the plane. The visibility parameter of map 50D has been set so that the map and its elements are locally transparent. Map 50D comprises lines of latitude and longitude, and because of the local transparency of the map the rear sections of the lines are visible, as well as the front sections. However, since map 50D is opaque with respect to map 50E, because of the local transparency of map 50D, there are no diamond shapes visible in sections 120, 122 of map 50D.

It will be appreciated that charts other than those described above, with elements having other relative visibilities, may be implemented as embodiments of the present invention. For example, ablation sites 52C4 and 52C5 (Fig. 3) may be added to chart 54C (Fig. 5) by appropriate definition of the visibility parameter of ablation sites 52C. Such a definition may incorporate, for example, a region of the chart wherein ablation sites are to be rendered visible, and/or a region wherein ablation sites are not to be visible. Alternatively or additionally, the visibility parameter may include a time component. For example, ablation sites which have been produced within a predefined time range of a procedure are rendered visible, but may or may not be visible outside the range, typically depending on a choice made by professional 28.

The description above has referred to forming a chart from two maps, by assigning a visibility parameter to elements of at least one of the maps. Those having ordinary skill in the art will be able to adapt the description to form the chart from three or more maps, while assigning a visibility parameter to elements of at least one of the maps.

It will thus be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

## Claims

1. Apparatus for mapping a body organ, comprising:
a processor which is configured to:
receive a three-dimensional (3D) map of the body organ together with items of auxiliary information having respective location coordinates in a frame of reference of the 3D map,
apportion the items into a plurality of sub-groups, and
assign to a selected sub-group a visibility parameter indicative of a relative visibility of the selected sub-group in relation to the map and to other sub-groups; and
a screen, coupled to the processor, which is configured to display the 3D map of the body organ in a selected orientation while the processor selectively superimposes on the 3D map one or more of the items in the selected sub-group responsively to the orientation, the respective location coordinates of the items, and the assigned visibility parameter.

2. A method for mapping a body organ, comprising:
receiving a three-dimensional (3D) map of the body organ together with items of auxiliary information having respective location coordinates in a frame of reference of the 3D map;
apportioning the items into a plurality of sub-groups;
assigning to a selected sub-group a visibility parameter indicative of a relative visibility of the selected sub-group in relation to the map and to other sub-groups; and
displaying the 3D map of the body organ in a selected orientation while selectively superimposing on the 3D map one or more of the items in the selected sub-group responsively to the orientation, the respective location coordinates of the items, and the assigned visibility parameter.

3. The apparatus according to claim 1 or the method according to claim 2, wherein the items of auxiliary information comprise a further 3D map of a portion of the body organ, and wherein the further 3D map is assigned a further-3D-map visibility parameter.

4. The apparatus or the method according to claim 3, wherein the further-3D-map visibility parameter causes the further 3D map to be locally transparent, so that all elements of the further 3D map are visible while the further 3D map is opaque with respect to the 3D map.

5. The apparatus or the method according to claim 3, wherein the further 3D map is disjoint from the 3D map, or intersects the 3D map.

6. The apparatus according to claim 1 or the method according to claim 2, wherein the body organ comprises a heart, and wherein the selected sub-group comprises local activation times (LATs) of the heart.

7. The apparatus or the method according to claim 6, wherein the LATs comprise measured LATs.

8. The apparatus or the method according to claim 7, wherein the LATs comprise interpolated LATs derived from the measured LATs.

9. The apparatus according to claim 1 or the method according to claim 2, wherein the relative visibility comprises a transparency of the selected sub-group, or at least one of a color and a shading applied to the selected sub-group.

10. The apparatus according to claim 1 or the method according to claim 2, wherein the sub-groups are selected from a set comprising an ablation site, a catheter type, and a catheter measurement.

11. The apparatus according to claim 1 or the method according to claim 2, wherein the relative visibility of an element in the selected sub-group is a function of the location coordinates of the element, is a function of a proximity of the element to another element in the sub-group, is a function of a proximity of the element to another element in the other sub-groups, or is a function of a time of the mapping of the body organ.
